# EUROPEAN PATENT SPECIFICATION

(11) **EP 2 869 775 B1**
(45) Date of publication and mention of the grant of the patent: **18.04.2018**
(21) Application number: 13812499.5
(22) Date of filing: 12.06.2013
(51) Int. Cl.: A61B 17/88, A61B 17/86, A61F 2/32, A61F 2/46

(54) **SACRO-ILIAC JOINT IMPLANT SYSTEM**
KREUZ-DARMBEIN-GELENKIMPLANTATSYSTEM
SYSTÈME D'IMPLANT D'ARTICULATION SACRO-ILIAQUE

(30) Priority: 05.07.2012 US 201213542172
(43) Date of publication of application: 13.05.2015
(73) Proprietor: Warsaw Orthopedic, Inc., Warsaw, IN 46582 (US)
(72) Inventor: BECK, MD, Carter E., Missoula, MT 59804 (US); MARIK, Gregory C., Collierville, TN 38017 (US); METCALF, Newton H., Memphis, TN 38111 (US); WATT, Andrea T., Cordova, TN 38018 (US)
(74) Representative: Viering, Jentschura & Partner mbB Patent- und Rechtsanwälte
(86) International application number: PCT/US2013/045335
(87) International publication number: WO 2014/007953

(56) References cited:
- WO-A1-2011/063240
- GB-A- 2 479 829
- US-A- 6 053 916
- US-A1- 2007 270 879
- US-A1- 2010 087 878

## Description

### TECHNICAL FIELD

The present disclosure generally relates to medical devices for the treatment of musculoskeletal disorders, and more particularly to a surgical system for treating a sacro-iliac joint.

### BACKGROUND

The sacro-iliac joint is a a joint that joins the sacrum to the ilium bones of the pelvis. In the sacro-iliac joint, the sacral surface has hyaline cartilage that moves against fibrocartilage of the iliac surface. The spinal column is configured so that the weight of an upper body rests on the sacro-iliac joints at the juncture of the sacrum and ilia. Stress placed on the sacro-iliac joints in an upright position of the body makes the lower back susceptible to injury. Disorders of the sacro-iliac joint can cause low back and radiating buttock and leg pain in patients suffering from degeneration and laxity of the sacro-iliac joint. In some cases, the sacro-iliac joint can undergo dehydration and destabilization, similar to other cartilaginous joints, which causes significant pain. The sacro-iliac joint is also susceptible to trauma and degeneration, from fracture and instability. It is estimated that disorders of the sacro-iliac joint are a source of pain for millions of people suffering from back and radicular symptoms. Non-surgical treatments, such as medication, injection, mobilization, rehabilitation and exercise can be effective, however, may fail to relieve the symptoms associated with these disorders. Surgical treatment of these disorders includes stabilization and/or arthrodesis. Stabilization can include the use of implants, such as, for example, bone screws that are fixed with bone. Arthrodesis may include immobilization of a joint. The present disclosure describes an improvement over these prior art technologies.

A surgical system for treating a sacro-iliac joint is disclosed in US 2007/270879 A1.

### SUMMARY

According to the present invention, a surgical system for treating a sacro-iliac joint as defined in claim 1 is provided. Examples of such a system are defined in the dependent claims. The methods not covered by the claims represent background art that is useful for understanding the invention.

In one embodiment not covered by the present invention, a method for treating a sacro-iliac joint is provided. The method comprising the steps of: identifying a target of a posterior superior iliac spine of a body; determining a selected trajectory that includes the target and at least a portion of a sacrum of the body; creating a pathway in the body along the selected trajectory from a posterior approach to the body; and delivering an implant along the pathway such that the implant is disposed for fixation with an ilium of the body and the sacrum.

In one embodiment not covered by the present invention, a method for treating a sacro-iliac joint is provided. The method comprising the steps of: identifying a target of a posterior superior iliac spine of a body, the body having an ilium defining a first cortical layer and a second cortical layer, and a sacrum defining a first cortical layer and a second cortical layer; creating an incision in the body adjacent the posterior superior iliac spine; disposing a guidewire at the target; advancing the guidewire from the target along the sacro-iliac joint and into the sacrum along a selected trajectory; confirming the selected trajectory with medical imaging; creating a pathway along the selected trajectory from a posterior approach to the body, the pathway extending from at least the target through the cortical layers of the ilium and the first cortical layer of the sacrum; and delivering an implant along the pathway such that the implant extends through the second cortical layer of the ilium and the first cortical layer of the sacrum and is disposed for fixation with the ilium and the sacrum.

In one embodiment not covered by the present invention, a method for treating a sacro-iliac joint is provided. The method comprising the steps of: disposing a body in a prone position on a surface; identifying a target of a posterior superior iliac spine of the body, wherein the body defines a vertical axis extending from the target and the selected trajectory is disposed along a transverse axis relative to the vertical axis, the body having an ilium defining a first cortical layer and a second cortical layer, and a sacrum defining a first cortical layer and a second cortical layer; creating an incision in the body adjacent the posterior superior iliac spine; disposing a guidewire at the target; advancing the guidewire from the target along the sacro-iliac joint on an iliac side of the sacro-iliac joint and into the sacrum along a selected trajectory; confirming the selected trajectory with medical imaging; removing the guidewire from the pathway; creating a pathway along the selected trajectory with a reamer from a posterior approach to the body, the pathway extending from at least the target through the cortical layers of the ilium and the first cortical layer of the sacrum; disposing bone graft adjacent the pathway between the ilium and the sacrum; and delivering a screw, which extends between a leading end and a trailing end including a head having a planar surface, along the pathway such that the screw extends through the second cortical layer of the ilium and the first cortical layer of the sacrum and is disposed for fixation with the ilium and the sacrum, wherein the planar surface is disposed in substantially flush alignment with an outer surface of the ilium.

### BRIEF DESCRIPTION OF THE DRAWINGS

The present disclosure will become more readily apparent from the specific description accompanied by the following drawings, in which:
FIG. 1 is a perspective view of one particular embodiment of a component of a system in accordance with the principles of the present disclosure;
FIG. 2 is a side view of the component shown in FIG. 1;
FIG. 3 is an end view of the component shown in FIG. 1;
FIG. 4 is a cross-section view taken along lines A-A shown in FIG. 2;
FIG. 5 is an enlarged side view of detail A shown in FIG. 4;
FIG. 6 is a perspective view of one embodiment of components of a system in accordance with the principles of the present disclosure disposed with a sacro-iliac region of a body;
FIG. 7 is a breakaway view of the components and sacro-iliac region shown in FIG. 6;
FIG. 8 is a perspective view of one embodiment of components of a system in accordance with the principles of the present disclosure disposed with a sacro-iliac region of a body;
FIG. 9 is a breakaway view of components of the system and sacro-iliac region shown in FIG. 8;
FIG. 10 is an enlarged breakaway view of components of the system and sacroiliac region shown in FIG. 8;

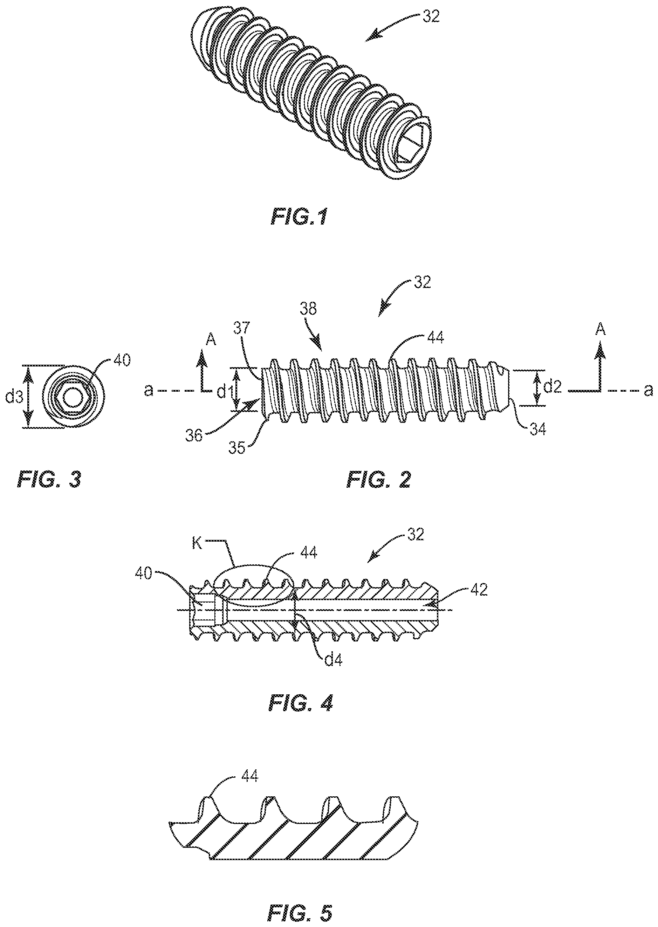

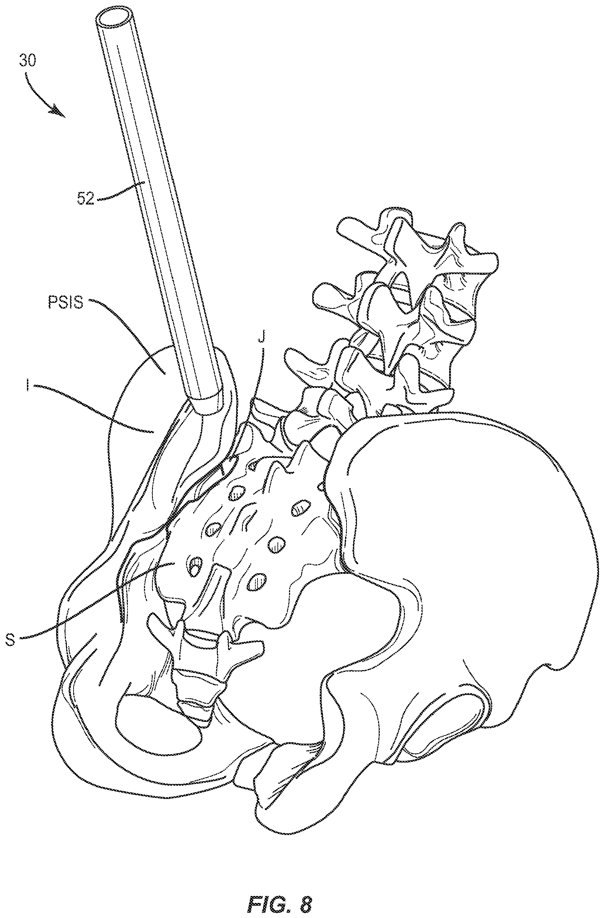

FIG. 1 1 is an enlarged breakaway view of components of the system and sacroiliac region shown in FIG. 8;

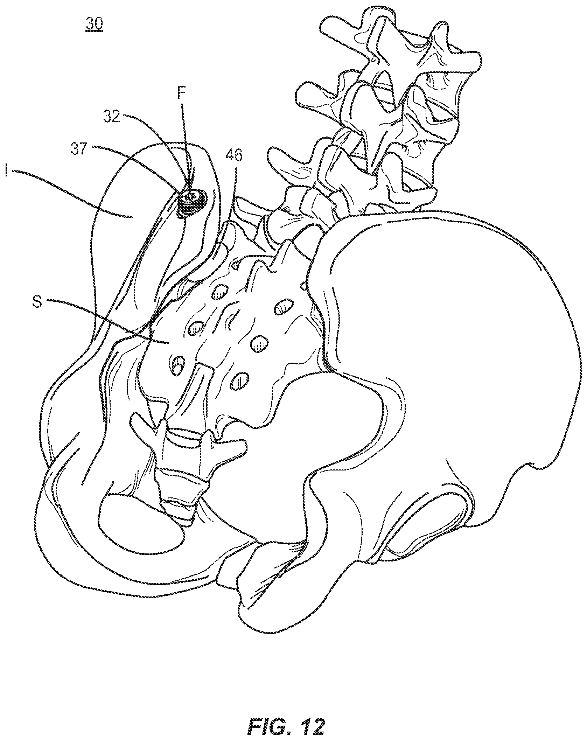

FIG. 12 is an enlarged breakaway view of components of the system and sacroiliac region shown in FIG. 8;

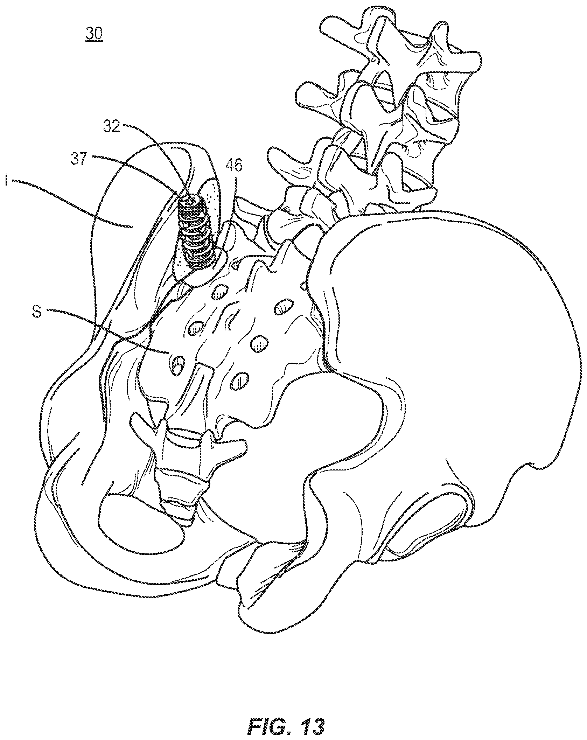

FIG. 13 is an enlarged breakaway view of the system and sacro-iliac region shown in FIG. 12;

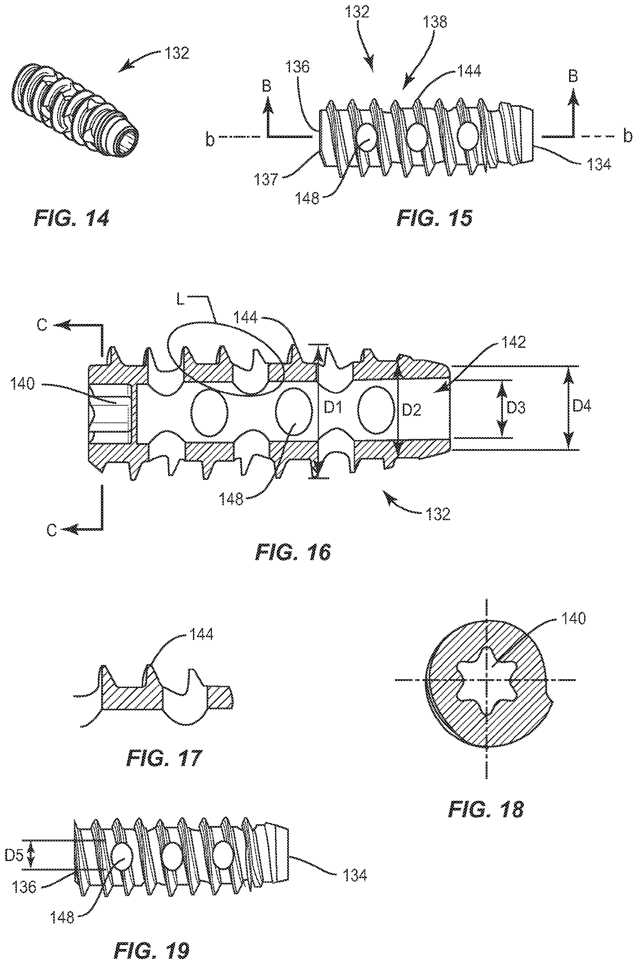

FIG. 14 is a perspective view of one embodiment of a component of a system accordance with the principles of the present disclosure;
FIG. 15 is a side view of the component shown in FIG. 14;
FIG. 16 is a cross-section view taken along lines B-B shown in FIG. 15;
FIG. 17 is an enlarged view of detail B shown in FIG. 16;
FIG. 18 is a cross-section view taken along lines C-C shown in FIG. 16; and
FIG. 19 is a side view of the component shown in FIG. 14.

### DETAILED DESCRIPTION

The exemplary embodiments of the surgical system disclosed are discussed in terms of medical devices for the treatment of musculoskeletal disorders and more particularly, in terms of a surgical system for treating the sacro-iliac (SI) joint. It is envisioned that the surgical system disclosed provide stability and maintains structural integrity while reducing stress on the SI joint. It is further envisioned that the present disclosure may be employed to treat musculoskeletal disorders including SI dysfunction or syndrome, dehydration, destabilization and/or laxity.

In one embodiment not covered by the present invention, the method includes identifying the posterior superior iliac spine on a patient that is positioned in a prone position on an operating table. The posterior superior iliac spine is used as a landmark for making an incision. It is contemplated that identification of the posterior superior iliac spine limits vascular and muscular disruption from a surgical approach. A trajectory path is established using fluoroscopy and a guide wire is inserted into the posterior superior iliac spine, for example, on an iliac side of a SI joint. The guide wire is advanced across the SI joint into the sacrum. Upon breaching the sacral side of the SI joint, the advancement of the guidewire is stopped.

In one embodiment not covered by the present invention, the trajectory is confirmed by confirming guidewire placement with medical imaging, such as, for example, fluoroscopy. In one embodiment not covered by the present invention, the guide wire is passed through the ilium, into the first cortical margin of the sacrum and through the SI joint. It is envisioned that neural structures that exit the posterior of the sacrum are avoided. In one embodiment not covered by the present invention, a guide tube is placed over the guidewire according to the trajectory path of the guidewire above the posterior superior iliac spine of the patient. In one embodiment, the guide wire is removed. A reamer can be inserted over the guidewire or inserted over the guide tube. A surgical pathway is reamed through the ilium, across the SI joint and through the first cortical margin of the sacrum. In one embodiment not covered by the present invention, the reamer prepares an implant space along the trajectory path determined with the guidewire. In one embodiment not covered by the present invention, placement is confirmed with medical imaging.

In one embodiment not covered by the present invention, bone graft material, such as, for example, autograft and/or allograft is inserted into the SI joint space to create a bony contact between the iliac and sacrum sides. In one embodiment not covered by the present invention, the bone graft material is inserted into a cannula of a screw.

In one embodiment not covered by the present invention, a SI fixation screw is attached to a driver. A downward force is applied and the screw is driven through the ilium, through the graft material and into the sacrum following the path created by the reamer until the screw is flush with the ilium and docked into the sacrum. In one embodiment not covered by the present invention, screw placement is confirmed with fluoroscopy and the incision is closed.

In one embodiment not covered by the present invention, a method is provided for screw removal from the SI joint fusion. In one embodiment not covered by the present invention, the method includes providing an implant inserter configured to attach to the screw. The iliac side of the SI joint of a patient who underwent a SI fusion procedure is exposed. In one embodiment not covered by the present invention, a tube can be placed over the incision site.

The dorsal aspect of the screw is positively identified. In one embodiment not covered by the present invention, the dorsal aspect of the screw is identified by fluoroscopy. The implant inserter is re-attached to the dorsal end of the screw and the screw is removed.

It is contemplated that one or all of the components of the surgical system may be disposable, peel-pack, pre-packed sterile devices. One or all of the components of the system may be reusable. The system may be configured as a kit with multiple sized and configured components.

It is envisioned that the present disclosure may be employed to treat spinal disorders that may include, but are not limited to, sacro-iliac joint disruptions, degenerative sacroilitis, degenerative disc disease, disc herniation, osteoporosis, spondylolisthesis, stenosis, scoliosis and other curvature abnormalities, kyphosis, tumor and fractures. It is contemplated that the present disclosure may be employed with other osteal and bone related applications, including those associated with diagnostics and therapeutics. It is further contemplated that the disclosed system may be alternatively employed in a surgical treatment with a patient in a prone or supine position, and/or employ various surgical approaches to the spine, including anterior, posterior, posterior mid-line, direct lateral, postero-lateral, and/or antero-lateral approaches, and in other body regions. The present disclosure may also be alternatively employed with procedures for treating the lumbar, cervical, thoracic and pelvic regions of a spinal column. The system of the present disclosure may also be used on animals, bone models and other non-living substrates, such as, for example, in training, testing and demonstration.

The present disclosure may be understood more readily by reference to the following detailed description of the disclosure taken in connection with the accompanying drawing figures, which form a part of this disclosure. It is to be understood that this disclosure is not limited to the specific devices, methods, conditions or parameters described and/or shown herein, and that the terminology used herein is for the purpose of describing particular embodiments by way of example only and is not intended to be limiting of the claimed disclosure. Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior".

Further, as used in the specification and including the appended claims, "treating" or "treatment" of a disease or condition refers to performing a procedure that may include administering one or more drugs to a patient (human, normal or otherwise or other mammal), in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (e.g., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, e.g., arresting its development, or relieving the disease, e.g., causing regression of the disease. For example, treatment can include reducing acute or chronic inflammation; alleviating pain and mitigating and inducing re-growth of new ligament, bone and other tissues; as an adjunct in surgery; and/or any repair procedure. Also, as used in the specification and including the appended claims, the term "tissue" includes soft tissue, ligaments, tendons, cartilage and/or bone unless specifically referred to otherwise.

The following discussion includes a description of a surgical system in accordance with the principles of the present disclosure. Alternate embodiments are also disclosed. Reference will now be made in detail to the exemplary embodiments of the present disclosure, which are illustrated in the accompanying figures. Turning now to FIGS. 1-5, there are illustrated components of a surgical system, such as, for example, a SI implant system 30 in accordance with the principles of the present disclosure.

The components of system 30 can be fabricated from biologically acceptable materials suitable for medical applications, including metals, synthetic polymers, ceramics, bone material, tissue and/or their composites, depending on the particular application and/or preference of a medical practitioner. For example, the components of system 30, individually or collectively, can be fabricated from materials such as stainless steel alloys, commercially pure titanium, titanium alloys, Grade 5 titanium, super-elastic titanium alloys, cobalt-chrome alloys, stainless steel alloys, superelastic metallic alloys (e.g., Nitinol, super elasto-plastic metals, such as GUM METAL® manufactured by Toyota Material Incorporated of Japan), ceramics and composites thereof such as calcium phosphate (e.g., SKELITETM manufactured by Biologix Inc.), thermoplastics such as polyaryletherketone (PAEK) including polyetheretherketone (PEEK), polyetherketoneketone (PEKK) and polyetherketone (PEK), carbon-PEEK composites, PEEK-BaS04 polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri- calcium phosphate (TCP), hydroxyapatite (HA)-TCP, calcium sulfate, or other resorbable polymers such as polyaetide, polyglycolide, polytyrosine carbonate, polycaroplaetohe and their combinations. Various components of system 30 may have material composites, including the above materials, to achieve various desired characteristics such as strength, rigidity, elasticity, compliance, biomechanical performance, durability and radiolucency or imaging preference. The components of system 30, individually or collectively, may also be fabricated from a heterogeneous material such as a combination of two or more of the above-described materials. The components of system 30 may be monolithically formed, integrally connected or include fastening elements and/or instruments, as described herein.

System 30 includes an orthopedic implant, such as, for example, a screw 32, as shown in FIG. 1. Screw 32 is cannulated. Screw 32 is configured to assist in the treatment of SI joint disorders including those caused by degeneration or trauma. It is contemplated that screw 32 may be employed for arthrodesis applications, as will be described.

Screw 32 includes a leading end 34, which is tapered. It is contemplated that end 34 is tapered, for example, with a bevel for easier insertion and less tearing of the tissue, such as the cortical layers of the ilium and the sacrum. Screw 32 includes a trailing end 36, as shown in FIG. 2. End 36 includes a head 35 having a planar surface 37. An elongated shaft 38 is disposed along a longitudinal axis a. Shaft 38 has a cylindrical cross- section configuration and includes an outer surface having a continuous screw thread 44 formed thereon. It is contemplated that the length of shaft 38 can vary from about 20 millimeters (mm) to about 60 mm. It is further contemplated that an engaging structure may be located on shaft 38, such as, for example, a nail configuration, barbs, expanding elements, raised elements and/or spikes to facilitate engagement of shaft 38 with tissue, such as, for example, bone and adjacent tissue.

Screw 32 includes a diameter dl adjacent end 36. It is envisioned that diameter dl can vary in size from about 3.5 mm to about 7.00 mm. End 34 is tapered to a diameter d2 in a configuration to facilitate penetration with tissue. It is envisioned that diameter d2 can vary in size from about 2.8 mm to about 5.7 mm. Screw 32 includes a socket 40 at end 36. Socket 40 is hexagonal. Socket 40 is configured to accommodate a driver (not shown), which attaches to screw 32 so that screw 32 can be driven into an implant space prepared by a medical device, such as for example, a reamer, as described herein.

Screw 32 includes a major diameter d3, as shown in FIG. 3. It is contemplated that diameter d3 can vary in size from about 5.0 mm to about 8.5 mm. Screw 32 has a uniform minor diameter d4, as shown in FIG. 4. It is contemplated that diameter d4 can vary from about 3.5 mm to about 6.4 mm. Screw 32 defines a hollow central shaft, such as, for example, cannulated portion 42. Cannulated portion 42 is configured to facilitate passage of a guide wire and/or bone graft and/or agents, as described herein.

System 30 includes an orthopedic implant, such as, for example, bone graft 46. Graft 46 is configured for disposal and engagement on or about the surfaces of screw 32 and/or anatomical surfaces of a body. It is envisioned that graft 46 may be configured as and/or include one or a plurality of a fastener, screw, cage, spinal rod and/or connector. It is further envisioned that graft 46 may be variously configured including cylindrical, rectangular, oval, uniform, non-uniform, mesh, staggered and/or undulating. In one embodiment, graft 46 includes an agent, which may be disposed, packed or layered within, on or about the surfaces of an implant, such as, screw 32 and/or a selected portion of an anatomy.

It is envisioned that the agent may include bone growth promoting material, such as, for example, bone graft to enhance fixation of the fixation elements with vertebrae V. Osteogenic material may be included in the agent such as, for example, autologous bone harvested from the patient receiving the implant device, bone allograft, bone xenograft, any number of non-bone implants (for example ceramic, metallic, polymer), bone morphogenic protein, and/or bio-resorbable compositions. For example, the osteogenic material may comprise minerals such as calcium phosphate or calcium sulfate minerals, bone, including xenograft, allograft or autograft bone. The osteogenic material may also comprise demineralized bone matrix (DBM), osteoinductive factors such as bone morphogenetic proteins (for example human BMP-2 or human BMP-7 or heterodimers thereof) whether recombinantly produced or purified from tissues, LIM mineralization proteins (LMPs), or the like. The osteogenic material may also comprise a binder material such as blood, clottable blood fractions, platelet gel, collagen, gelatin, carboxymethyl cellulose, or other similar materials that will serve to bind together harder particles or materials such as mineral particles (for example bone or synthetic mineral particles) so as to create a three-dimensionally stable mass when compacted into the cavities of the implant device.

Graft 46 can contain other bioactive agents or other active agents, which may include one or a plurality of therapeutic agents and/or pharmacological agents for release, including sustained release, into the SI joint to treat, for example, pain, inflammation and degeneration. The agents may include pharmacological agents, such as, for example, antibiotics, pain medications, analgesics, anesthetics, anti-inflammatory drugs including but not limited to steroids, anti-viral and anti-retroviral compounds, therapeutic proteins or peptides, therapeutic nucleic acids (as naked plasmid or a component of an integrating or non- integrating gene therapy vector system), and combinations thereof.

The agent may also include analgesics or anesthetics such as acetic acid derivatives, clonidine, COX-2 selective inhibitors, COX-2 inhibitors, enolic acid derivatives, propionic acid derivatives, salicylic acid derivatives, opioids, opioid/nonopioid combination products, adjuvant analgesics, and general and regional/local anesthetics.

The agent may also include antibiotics such as, for example, amoxicillin, beta-lactamases, aminoglycosides, beta-lactam (glycopeptide), clindamycin, chloramphenicol, cephalosporins, ciprofloxacin, erythromycin, fluoroquinolones, macrolides, metronidazole, penicillins, quinolones, rapamycin, rifampin, streptomycin, sulfonamide, tetracyclines, trimethoprim, trimethoprim-sulfamthoxazole, and vancomycin.

The active agent may also include immunosuppressives agents, such as, for example, steroids, cyclosporine, cyclosporine analogs, cyclophosphamide, methylprednisone, prednisone, azathioprine, FK-506, 15-deoxyspergualin, prednisolone, methotrexate, thalidomide, methoxsalen, rapamycin, leflunomide, mizoribine (bredinin™), brequinar, deoxyspergualin, and azaspirane (SKF 105685), Orthoclone OKT.™ 3 (muromonab-CD3). Sandimmune™, Neoral™, Sangdya™ (cyclosporine), Prograf™ (FK506, tacrolimus), Cellcept™ (mycophenolate motefil, of which the active metabolite is mycophenolic acid), Imuran™ (azathioprine), glucocorticosteroids, adrenocortical steroids such as Deltasone™ (prednisone) and Hydeltrasol™ (prednisolone), Folex™ and Mexate™ (methotrxate), Oxsoralen-Ultra™ (methoxsalen) and Rapamune™ (sirolimus).

System 30 may include radio markers for identification of one or more of the components of system 30 under x-ray, fluoroscopy, CT or other medical imaging techniques. Metallic or ceramic radiomarkers, such as tantalum beads, tantalum pins, titanium pins, titanium endcaps and platinum wires can be used.

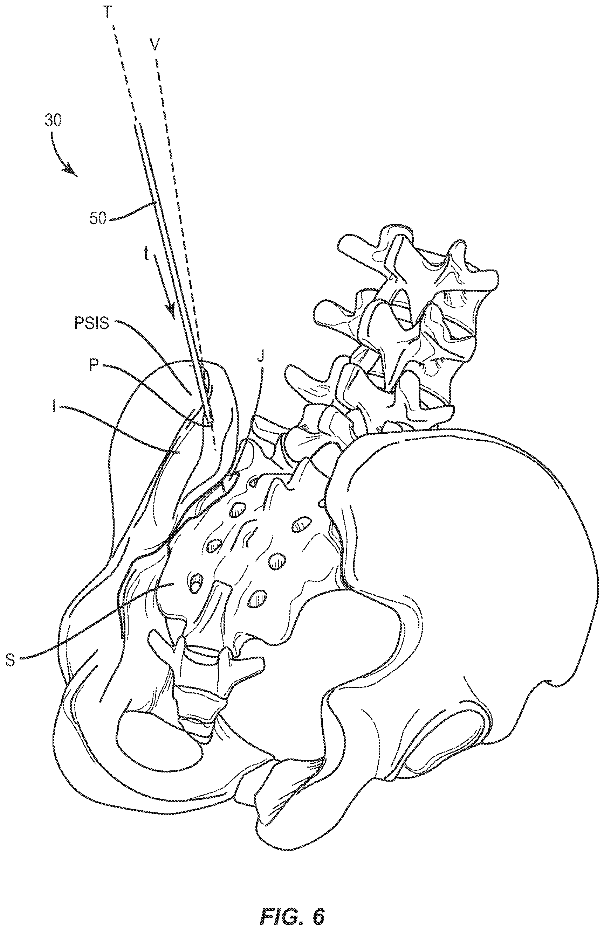

In assembly, operation and use, system 30, similar to that described, is employed with a surgical procedure for treatment of a SI joint J of a patient. System 30 may also be employed with other surgical procedures. For example, system 30 is employed with a surgical arthrodesis procedure, such as, for example, fusion for treatment of an applicable condition or injury of an affected SI joint J, as shown in FIGS. 6-13. It is contemplated that components of system 30 are inserted with SI joint J to space apart articular joint surfaces, establish joint tension, provide support and maximize stabilization of sacro-iliac joint J. It is further contemplated that the components of system 30 are inserted with a SI joint J as a SI joint spacer to restore ligamentous tension, eliminate painful micro-motion, and/or separate and cushion opposing articulating surfaces that cause pain. It is envisioned that system 30 may maintain joint tension without promoting bone growth.

It is envisioned that system 30 may be used in any existing surgical method or technique including open surgery, mini-open surgery, minimally invasive surgery including percutaneous surgical implantation, whereby SI joint J is accessed through a mini-incision, or sleeve that provides a protected passageway to the area. Once access to the target site is obtained, the particular surgical procedure is performed for treating the SI joint disorder. System 30 is then employed to augment the surgical treatment. System 30 can be delivered or implanted as a pre-assembled device or can be assembled in situ. System 30 may be completely or partially revised, removed or replaced in situ. It is contemplated that one or all of the components of system 30 can be delivered to the surgical site via manual manipulation and/or a free hand technique.

In use, to treat the affected section of SI joint J, the body of a patient is disposed in a prone position on a surface, such as, for example, a surgical table (not shown). A target P of a posterior superior iliac spine (PSIS) of the body is identified, as shown in FIG. 6. In one embodiment not covered by the present invention, the PSIS is identified through manipulation or tactile feedback by touching the skin of the patient. In one embodiment not covered by the present invention, the PSIS is identified through medical imaging, such as, for example, x-ray and/or fluoroscopy. It is envisioned that identifying target P via the PSIS approach to SI fusion can help limit vascular and muscular disruption during the surgical procedure.

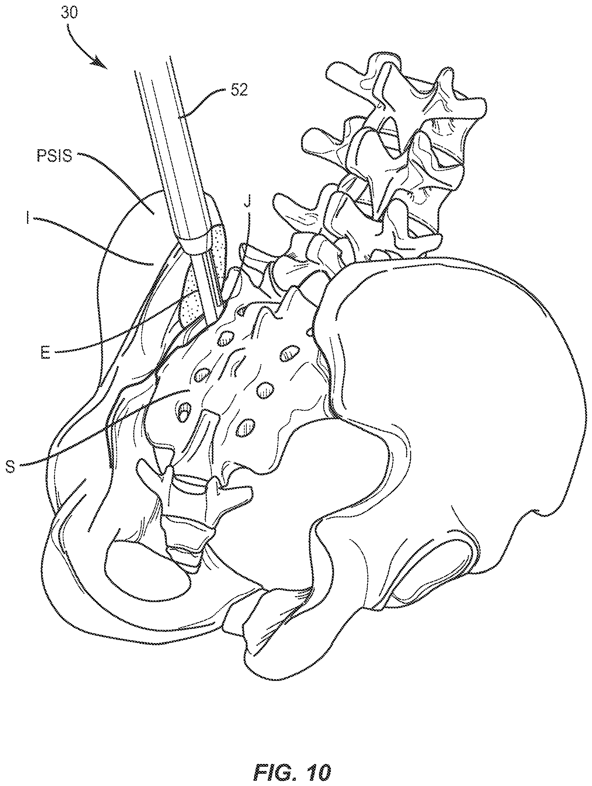

The body defines a vertical axis V extending from target P. A selected trajectory, such as, for example, a PSIS trajectory t is disposed along a transverse axis T relative to axis V to define a surgical pathway E (FIG. 10) for introduction and/or delivery of components of system 30 to the surgical site. The body has an ilium I that defines a first cortical layer and a second cortical layer, and cancellous bone disposed therebetween. A sacrum S that defines a first cortical layer and a second cortical layer, and cancellous bone disposed therebetween. It is contemplated that a medical practitioner obtains access to the PSIS target P including SI joint J in any appropriate manner, such as through incision and retraction of tissues.

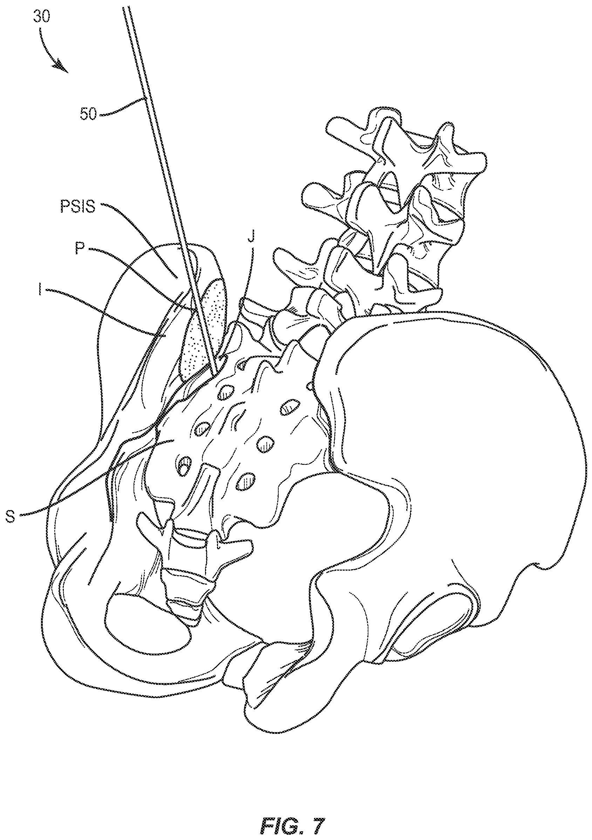

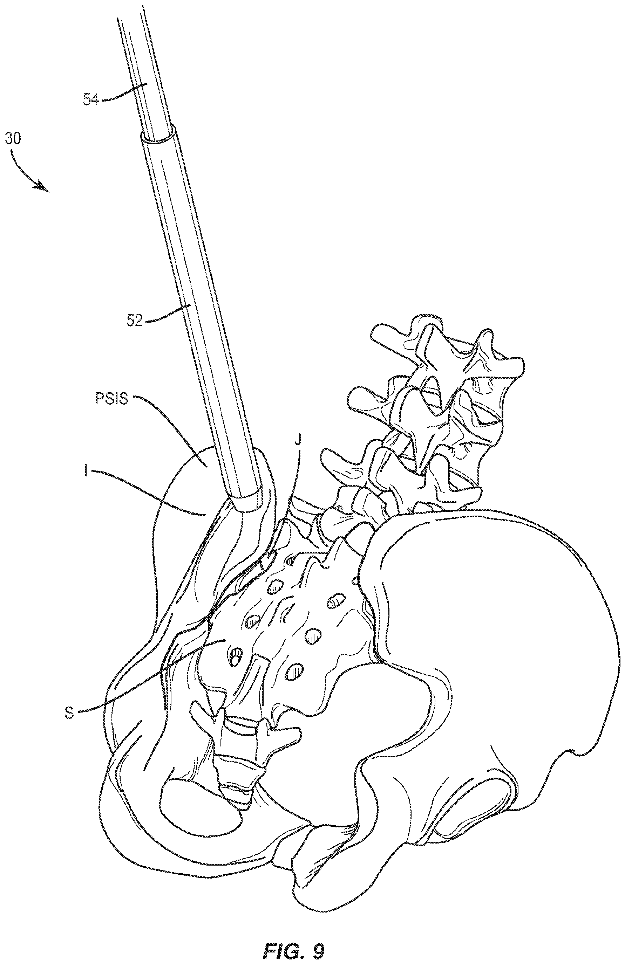

A guidewire, such as, for example, a K-wire 50 is introduced at target P, as shown in FIG. 7. In one embodiment not covered by the present invention, a working channel such as a guide tube 52 is introduced to surgical pathway E defined by trajectory t and placed over guide wire 50, as shown in FIG. 8. Tube 52 is anchored on a top surface of ilium I adjacent the PSIS. K-wire 50 is advanced via an insertion needle or a similar instrument from target P along trajectory t of surgical pathway E through the cortical layers and the cancellous bone of ilium I into SI joint J and into the first cortical layer of sacrum S, as shown in FIGS. 9 and 10.

K-wire 50 advancement is stopped after breaching the first cortical layer of sacrum S. In one embodiment not covered by the present invention, K-wire 50 is advanced through the first cortical layer of sacrum S only. It is envisioned that K-wire 50 may extend in the cancellous bone of sacrum S. It is further envisioned that this configured avoids neural structures disposed adjacent posterior of sacrum S. In one embodiment not covered by the present invention, a selected trajectory, such as, for example, a PSIS trajectory is disposed approximately 15 degrees lateral or vertical relative to vertical axis V. In one embodiment not covered by the present invention, trajectory t and/or surgical pathway E is confirmed with medical imaging. In one embodiment not covered by the present invention, trajectory t and/or surgical pathway E is confirmed with C-arm or O-arm fluoroscopy.

It is envisioned that trajectory t and/or surgical pathway E may be confirmed with alternative imaging modality or image-less based application. For example, it is contemplated that medical imaging in accordance with the present disclosure may include isocentric fluoroscopy, bi-plane fluoroscopy, ultrasound, computed tomography, multi-slice computed tomography, magnetic resonance imaging, high frequency ultrasound, optical coherence tomography, intra-vascular ultrasound, 2D, 3D or 4D ultrasound, intraoperative CT, MRI, or O-arms having single or multi flat panels receivers that move about the ring to acquire fluoroscopic images may also be used to acquire pre-operative or real-time images or image data of the trajectory of various elements utilized in the fusion procedure described herein. It is further contemplated that image datasets from hybrid modalities, such as positron emission tomography combined with CT, or single photon emission computer tomography combined with CT, could also provide functional image data superimposed onto anatomical data to be used to confidently reach target sights within the areas of interest.

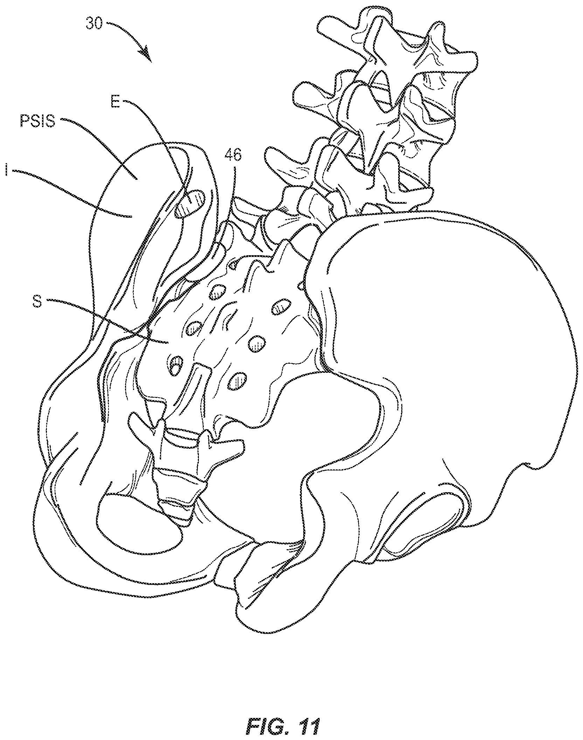

In one embodiment not covered by the present invention, K-wire 50 is removed from surgical pathway E. Pathway E is further defined along trajectory t with a reamer 54 from a posterior approach to the body. Pathway E is enlarged via reamer 54 from target P, through the cortical layers and the cancellous bone of ilium I into SI joint J and into the first cortical layer of sacrum S, as shown in FIG. 11. Pathway E includes a cavity configured for disposal of at least one implant, such as, for example, screw 32 and graft 46.

Graft 46 is introduced and/or delivered along pathway E to adjacent the surgical site and into the SI joint. Graft 46 may be disposed, packed and/or layered about the surfaces of ilium I and sacrum S. Graft 46 facilitates a bony contact between ilium I and sacrum S. In one embodiment not covered by the present invention, fusion may be facilitated or augmented by introducing or positioning graft 46 within cavities of an implant.

Screw 32 is introduced and/or delivered along pathway E to adjacent the surgical site and into the SI joint via a driver (not shown). A downward force, in the direction shown by arrow A in FIG. 11, is applied to drive screw 32 such that screw 32 extends through the cortical layers and the cancellous bone of ilium I into SI joint J, through graft 46 and into the first cortical layer of sacrum S, as shown in FIG. 12. Screw 32 is disposed for fixation with ilium I and sacrum S. Planar surface 37 of head 35 is disposed in substantially flush alignment with an outer surface of ilium I, as shown in FIG. 13. Upon completion of the procedure, the non-implant components of system 30 are removed from the surgical site and the incision is closed.

In one embodiment not covered by the present invention, a method is provided for the removal of screw 32. The body of a patient is disposed in a prone position on a surgical table. A target of a PSIS of the body is identified. A dorsal aspect of screw 32 is identified. In one embodiment not covered by the present invention, medical imaging is employed to confirm the identification of the dorsal aspect. A medical practitioner obtains access to a target including SI joint J, similar to that described above, in any appropriate manner, such as through incision and retraction of tissues. An implant inserter (not shown) is provided that is configured for attachment to screw 32. The iliac side of the sacro-iliac joint of the patient is exposed and the incision site is reopened. End 36 of screw 32 is identified. The implant inserter is attached to end 36. Screw 32 is removed and the incision site is closed.

In one embodiment not covered by the present invention, similar to the system and methods described above, a method for SI fusion is provided. The patient is positioned in a prone position. The PSIS is identified and an incision path is created. A trajectory is established with a guidewire and fluoroscopy is used to confirm trajectory. A working channel is placed over a guide wire. The guide wire is removed from the working channel. A reamer is inserted through the working channel along the trajectory path. The trajectory is confirmed with an imaging technique. Bone growth material is inserted into the SI joint. A cannulated screw is driven into the implant space. Positioning of the screw is verified with an imaging technique and the incision is closed.

In one embodiment, as shown in FIGS. 14-19, system 30, similar to the systems and methods described above with regard to FIGS. 1-13, includes an orthopedic implant, such as, for example, a screw 132, similar to screw 32 described above. Screw 132 is cannulated. Screw 132 includes a leading end 134, which is tapered. Screw 132 includes a trailing end 136. End 136 includes a head 135 having a planar surface 137. An elongated shaft 138 is disposed along a longitudinal axis b. Shaft 138 has a cylindrical cross-section configuration and includes an outer surface having a continuous screw thread 144 formed thereon. It is contemplated that the length of shaft 138 can vary from about 20 mm to about 60 mm.

Screw 132 includes a uniform major diameter Dl, as shown in FIG. 16. It is contemplated that diameter Dl can vary in size from about 11 mm to about 18 mm. Screw 132 includes a uniform minor diameter D2. It is contemplated that diameter D2 can vary in size from about 8 mm to about 15 mm.

Screw 132 includes a socket 140 at end 136. Socket 140 is hexagonal. Socket 140 is configured to accommodate a driver (not shown), which attaches to screw 132 so that screw 132 can be driven in an implant space prepared by a medical device, such as, for example, a reamer, as described herein.

Screw 132 defines a hollow central cavity, such as, for example, cannulated portion 142. Screw 132 includes uniform threads 144, as shown in FIG. 17. Cannula 142 has an inside diameter D3. It is envisioned that diameter D3 can vary in size from about 5 mm to about 10 mm. Screw 132 includes an outside diameter D4 at end 134. It is contemplated that diameter D4 can vary in size from about 7 mm to about 14 mm. Screw 132 includes at least one or more fenestrations, such as, for example, openings 148. Openings 148 are configured to communicate with cannulated portion 142 and are configured to disperse flowable materials, such as, for example, biologies, agents, medical adhesives, bonding cements and/or bone healing substances, similar to those described herein. In one embodiment, openings 148 are formed in close proximity to end 134. In one embodiment, openings 148 are formed in close proximity to end 136, as shown in FIG. 19. In one embodiment, openings 148 are oval and have a major diameter D5. It is contemplated that the size of diameter D5 can vary from about 2 mm to about 3.8 mm. It is further contemplated that the shape of holes 148 may be variously configured, such as, for example, round, square, rectangular and/or polygonal.

## Claims

1. A surgical system for treating a sacro-iliac joint (J) jointing sacrum (S) and ilium (I), the system comprising:
a set screw (32; 132) including a tapered leading end (34; 134), a trailing end (36; 136) with a planar surface (37; 137) and a socket (40; 140) which is configured to accommodate a driver so that the screw (32; 132) can be driven into an implant space prepared by a medical device, and an elongated shaft (38; 138) which is disposed along a longitudinal axis (a) between the leading end (34; 134) and the trailing end (36; 136), wherein the shaft (38; 138) has a cylindrical cross-section configuration and includes an outer surface having a continuous screw thread (44; 144) formed thereon, wherein the screw thread (44; 144) continuously extends between the trailing end (36; 136) and the leading end (34; 134) of the screw (32; 132) and is formed on a portion of the outer surface of the shaft (38; 138) surrounding the socket (40; 140), and wherein the screw (32; 132) is cannulated defining a cannulated portion (42; 142) configured to facilitate passage of a guide wire (50) and/or bone graft and/or agents, and
a bone graft (46) which is configured for disposal and engagement on or about the surfaces of the screw (32; 132) and anatomical surfaces of a body and to be disposed, packed and/or layered about the surfaces of the ilium (I) and the sacrum (S) to facilitate a bony contact between the ilium (I) and the sacrum (S), wherein
the shaft (38; 138) of the screw (32; 132) has a length which varies from about 20 mm to about 60 mm so that the screw (32; 132) can be disposed for fixation with the ilium (I) and the sacrum (S) of the sacro-iliac joint (J) in a cavity of a pathway (E) formed in the body along a selected trajectory that includes a target of a posterior superior iliac spine of the body and at least a portion of the sacrum (S) of the body in a manner such that the screw (32; 132) extends through cortical layers and cancellous bone of the ilium (I) into the sacro-iliac joint (J), through the bone graft (46) and into the first cortical layer of the sacrum (S) with the planar surface (37) being disposed in substantially flush alignment with an outer surface of the ilium (I)..

2. The system according claim 1, wherein the screw (32) includes a diameter (d1) adjacent end (36) which varies in size from about 3,5 mm to about 7,0 mm, wherein the end (34) is tapered to a diameter (d2) which varies in size from about 2,8 mm to about 5,7 mm, wherein the major diameter (d3) varies in size from about 5,0 mm to about 8,5 mm, and wherein the screw (32) has a uniform minor diameter (d4), which varies from about 3,5 mm to about 6,4 mm.

3. The system according claim 1 or 2, wherein the screw (132) includes at least one or more openings (148) configured to communicate with the cannulated portion (142) and to disperse flowable materials.

4. The system according claim 3, wherein the openings (148) are formed in close proximity to the leading end (134) or the trailing end (136).

5. The system according to anyone of the preceding claims, wherein the bone graft (46) is configured as and/or includes one or a plurality of a fastener, screw, cage, spinal rod and/or connector.

6. The system according to anyone of the preceding claims, wherein the bone graft (46) is configured to be cylindrical, rectangular, oval, uniform, non-uniform, a mesh, staggered and/or undulating.

7. The system according to anyone of the preceding claims, wherein the bone graft (46) includes an agent which is disposed, packed or layered within, on or about the surfaces of the screw (32) and/or a selected portion of an anatomy.

8. The system according to anyone of the preceding claims, wherein the system is configured as a kit with multiple sized and configured components.

## Patentansprüche

1. Chirurgisches System zum Behandeln eines Iliosakralgelenks (J), welches ein Kreuzbein (S) und ein Darmbein (I) verbindet, wobei das System aufweist:
eine Feststellschraube (32; 132), welche aufweist: ein sich verjüngendes vorauseilendes Ende (34; 134), ein nacheilendes Ende (36; 136) mit einer planaren Fläche (37; 137) und einem Sockel (40; 140), welcher zur Aufnahme eines Schlüssels eingerichtet ist, so dass die Schraube (32; 132) in einen durch eine medizinische Vorrichtung vorbereiteten Implantationsraum eingeführt werden kann, und einen länglichen Schaft (38; 138), welcher entlang einer Längsachse (a) zwischen dem vorauseilenden Ende (34; 134) und dem nacheilenden Ende (36; 136) angeordnet ist, wobei der Schaft (38; 138) eine Zylindrischer-Querschnitt-Konfiguration und eine Außenfläche aufweist, die ein an dieser gebildetes durchgehendes Schraubengewinde (44; 144) aufweist, wobei das Schraubengewinde (44; 144) sich durchgehend zwischen dem nacheilenden Ende (36; 136) und dem vorauseilenden Ende (34; 134) der Schraube (32; 132) erstreckt und an einem Abschnitt der Außenfläche des Schafts (38; 138) gebildet ist, welcher den Sockel (40; 140) umgibt, und wobei die Schraube (32; 132) durchbohrt ist und einen durchbohrten Abschnitt (42; 142) definiert, der dazu eingerichtet ist, einen Durchgang eines Führungsdrahts (50) oder/und von Knochentransplantat oder/und Mitteln zu ermöglichen, und
ein Knochentransplantat (46), welches dazu eingerichtet ist, an oder um die Flächen der Schraube (32; 132) und an anatomischen Flächen bzw. um anatomische Flächen eines Körpers angeordnet und in Eingriff gebracht zu werden und um die Flächen des Darmbeins (I) und des Kreuzbeins (S) angeordnet, gepackt oder/und geschichtet zu werden, um einen Knochenkontakt zwischen dem Darmbein (I) und dem Kreuzbein (S) zu ermöglichen, wobei
der Schaft (38; 138) der Schraube (32; 132) eine Länge aufweist, die von ungefähr 20 mm bis ungefähr 60 mm variiert, so dass die Schraube (32; 132) für eine Befestigung an dem Darmbein (I) und dem Kreuzbein (S) des Iliosakralgelenks (J) in einem Hohlraum eines Durchgangs (E) angeordnet werden kann, welcher in dem Körper entlang einer ausgewählten Trajektorie gebildet ist, welche eine Zielposition eines hinteren oberen Darmbeinknochens des Körpers und wenigstens einen Abschnitt des Kreuzbeins (S) des Körpers in einer Weise aufweist, dass sich die Schraube (32; 132) durch kortikale Schichten und die Spongiosa des Darmbeins (I) in das Iliosakralgelenk (J) durch das Knochentransplantat (46) und in die erste kortikale Schicht des Kreuzbeins (S) erstreckt, wobei die planare Fläche (37) an einer Außenfläche des Kreuzbeins (I) im Wesentlichen bündig angeordnet ist.

2. System nach Anspruch 1, wobei die Schraube (32) einen zu dem Ende (36) benachbarten Durchmesser (d1) aufweist, dessen Größe von ungefähr 3,5 mm bis ungefähr 7,0 mm variiert, wobei sich das Ende (34) zu einem Durchmesser (d2) verjüngt, dessen Größe von ungefähr 2,8 mm bis ungefähr 5,7 mm variiert, wobei eine Größe des größten Durchmessers (d3) von ungefähr 5,0 mm bis ungefähr 8,5 mm variiert und wobei die Schraube (32) einen gleichmäßigen kleinsten Durchmesser (d4) aufweist, welcher von ungefähr 3,5 mm bis ungefähr 6,4 mm variiert.

3. System nach Anspruch 1 oder 2, wobei die Schraube (132) wenigstens eine oder mehrere Öffnungen (148) aufweist, welche dazu eingerichtet sind, mit dem durchbohrten Abschnitt (142) in Verbindung zu stehen und fließfähige Materialien zu verteilen.

4. System nach Anspruch 3, wobei die Öffnungen (148) in enger Nachbarschaft zu dem vorauseilenden Ende (134) oder dem nacheilenden Ende (136) gebildet sind.

5. System nach einem der vorhergehenden Ansprüche, wobei das Knochentransplantat (46) als ein oder mehrere Befestigungselemente, eine oder mehrere Schrauben, ein oder mehrere Käfige, ein oder mehrere Spinalstäbe oder/und ein oder mehrere Verbindungselemente eingerichtet ist oder/und ein oder mehrere Befestigungselemente, eine oder mehrere Schrauben, einen oder mehrere Käfige, einen oder mehrere Spinalstäbe oder/und ein oder mehrere Verbindungselemente aufweist.

6. System nach einem der vorhergehenden Ansprüche, wobei das Knochentransplantat (46) dazu eingerichtet ist, zylindrisch, rechtwinklig, oval, gleichmäßig, ungleichmäßig, ein Netz, gestuft oder/und gewellt zu sein.

7. System nach einem der vorhergehenden Ansprüche, wobei das Knochentransplantat (46) ein Mittel aufweist, welches innerhalb der, an den oder um die Flächen der Schraube (32) oder/und eines ausgewählten Abschnitts eines Körperteils angeordnet, gepackt oder geschichtet ist.

8. System nach einem der vorhergehenden Ansprüche, wobei das System als ein Bausatz mit Komponenten mit mehreren Größen und Konfigurationen eingerichtet ist.

## Revendications

1. Système chirurgical pour traiter une articulation sacro-iliaque (J) faisant la jointure entre un sacrum (S) et un ilium (I), le système comprenant :
une vis de réglage (32 ; 132) comportant une extrémité avant conique (34 ; 134), une extrémité arrière (36 ; 136) avec une surface plane (37 ; 137) et une douille (40 ; 140) qui est conçue pour loger un tournevis de sorte que la vis (32 ; 132) peut être introduite dans un espace d'implantation préparé par un dispositif médical, et une tige allongée (38 ; 138) qui est disposée le long d'un axe longitudinal (a) entre l'extrémité avant (34 ; 134) et l'extrémité arrière (36 ; 136), où la tige (38 ; 138) a une configuration de section cylindrique et comporte une surface externe sur laquelle est formé un filet de vis continu (44 ; 144), où le filet de vis (44 ; 144) s'étend en continu entre l'extrémité arrière (36 ; 136) et l'extrémité avant (34 ; 134) de la vis (32 ; 132) et est formé sur une partie de la surface externe de la tige (38 ; 138) entourant la douille (40 ; 140), et où la vis (32 ; 132) est perforée, définissant une partie perforée (42 ; 142) conçue pour faciliter le passage d'un fil-guide (50) et/ou d'un greffon osseux et/ou d'agents, et
un greffon osseux (46) qui est conçu pour être disposé et mis en prise sur ou autour des surfaces de la vis (32 ; 132) et de surfaces anatomiques d'un corps et pour être disposé, tassé et/ou superposé autour des surfaces de l'ilium (I) et du sacrum (S) pour faciliter un contact osseux entre l'ilium (I) et le sacrum (S), dans lequel
la tige (38 ; 138) de la vis (32 ; 132) a une longueur qui varie d'environ 20 mm à environ 60 mm de sorte que la vis (32 ; 132) peut être disposée de manière à être fixée à l'ilium (I) et au sacrum (S) de l'articulation sacro-iliaque (J) dans une cavité d'un passage (E) formé dans le corps le long d'une trajectoire choisie qui comporte une cible d'une épine iliaque supérieure postérieure du corps et au moins une partie du sacrum (S) du corps d'une manière telle que la vis (32 ; 132) s'étend à travers des couches corticales et un os spongieux de l'ilium (I) jusqu'à pénétrer dans l'articulation sacro-iliaque (J), à travers le greffon osseux (46) et dans la première couche corticale du sacrum (S) avec la surface plane (37) disposée en alignement sensiblement parfait avec une surface externe de l'ilium (I).

2. Système selon la revendication 1, dans lequel la vis (32) comporte un diamètre (d1) à proximité de l'extrémité (36) qui varie en taille d'environ 3,5 mm à environ 7,0 mm, dans lequel l'extrémité (34) s'effile jusqu'à un diamètre (d2) qui varie en taille d'environ 2,8 mm à environ 5,7 mm, dans lequel le grand diamètre (d3) varie en taille d'environ 5,0 mm à environ 8,5 mm, et dans lequel la vis (32) a un petit diamètre uniforme (d4) qui varie d'environ 3,5 mm à environ 6,4 mm.

3. Système selon la revendication 1 ou 2, dans lequel la vis (132) comporte au moins une ou plusieurs ouvertures (148) configurées pour communiquer avec la partie perforée (142) et pour disperser des matières fluides.

4. Système selon la revendication 3, dans lequel les ouvertures (148) sont formées à proximité immédiate de l'extrémité avant (134) ou de l'extrémité arrière (136).

5. Système selon l'une quelconque des revendications précédentes, dans lequel le greffon osseux (46) se présente sous la forme d'une attache, d'une vis, d'une cage, d'une tige vertébrale et/ou d'un connecteur et/ou comporte un ou une pluralité de ces derniers.

6. Système selon l'une quelconque des revendications précédentes, dans lequel le greffon osseux (46) se présente sous une forme cylindrique, rectangulaire, ovale, uniforme, non uniforme, de treillis, en quinconce et/ou ondulée.

7. Système selon l'une quelconque des revendications précédentes, dans lequel le greffon osseux (46) comporte un agent qui est disposé, tassé et/ou superposé dans, sur ou autour des surfaces de la vis (32) et/ou d'une partie choisie d'une anatomie.

8. Système selon l'une quelconque des revendications précédentes, dans lequel le système se présente sous la forme d'un kit doté d'éléments de tailles et de formes multiples.
